# EUROPEAN PATENT APPLICATION

(11) **EP 1 722 231 A2**
(43) Date of publication of application: **15.11.2006**
(21) Application number: 06008522.2
(22) Date of filing: 25.04.2006
(51) Int. Cl.: G01N 33/569, G01N 33/50, G01N 33/68, G01N 1/30, C07K 16/28, C40B 40/00

(54) **Method for identification of somatic stem cells**

(30) Priority: 27.04.2005 US 675148 P
(71) Applicant: MPB MelTec Patent- und Beteiligungsgesellschaft mbH, 39120 Magdeburg (DE); Otto-Von-Guericke-Universität Magdeburg, 39120 Magdeburg (DE)
(72) Inventor: Pommer, Ansgar J., Dr., 39108 Magdeburg (DE); Philipsen, Lars, Dipl.-Ing., 39114 Magdeburg (DE); Bastian, Anja, 39128 Magdeburg (DE); Gollnick, Harald, Prof. Dr., 39130 Magdeburg (DE); Bonnekoh, Bernd, Prof. Dr., 39116 Magdeburg (DE); Böckelmann, Raik, Dr., 39108 Magdeburg (DE); Schubert, Walter, Dr., 39175 Biederitz (DE)
(74) Representative: Patentanwälte Hofstetter, Schurack & Skora

(57) **Abstract**

The invention relates to a method for identification of somatic stem cells, wherein the spatial arrangement of epitopes in a sample is captured by MELC technology for identifying the phenotype specific to the somatic stem cells. The invention moreover relates to a composition, a kit and a biochip comprising at least one antibody and/or ligand which can be employed for the performance of the method.

## Description

The invention relates to a method for identification of somatic stem cells. The invention moreover relates to a composition, a kit and a biochip comprising antibodies and/or ligands which can be employed for this method.

Such a method as well as such a composition, kit and biochip can be employed for conducting laboratory tests for identifying somatic stem cells in particular keratinocyte stem cells.

As already described in a publication of Honeycut, K. A., Koster, M. I. & Roop, D.R. "Genes involved in stem cell fate decisions and commitment to differentiation play a role in skin disease" in J. Investig. Dermatol. Symp. Proc. 9, 261-268 (2004) candidate keratinocyte stem cells have the phenotype Ki67⁻/CD71⁻/pan-CK⁺/CD29⁺/CD49d⁺. But it has not so far been possible to map the distribution of cells defined by such a complex phenotype in tissue sections.

The invention therefore is based on the object of providing an option for the reliable identification of somatic stem cells, especially keratinocyte stem cells.

This object according to the invention is solved by way of a method, a composition, a kit as well as a biochip with the features of the independent claims.

Further advantageous variations are defined in the subclaims.

The solution of the object is based on the knowledge that somatic stem cells can be identified by aid of the so-called Multiple Epitope Ligand Cartography (MELC) technology by identifying certain epitopes on the basis of tissue samples. The information resulting from conventional immuno-phenotyping techniques is restricted by a limited resolution with regard to the numbers of epitopes to be detected simultaneously in a given sample or tissue section. For this reason somatic stem cells up to now could only be identified and isolated through radioactive marking. As such radioactive markings as a matter of course cannot be performed on the human being, an identification and therefore also a subsequent biochemical and molecular characterization of the human somatic stem cells was not possible up to now. These problems are circumvented by the MELC technology described in detail in the patent documents US 6,150,173, US 6,924,115, US 6,974,675, DE 197 09 348, EP 0 810 428, EP 1 136 822, and EP 1 136 823 and to which reference is made herewith. Its principle involves the sequential automated incubation of a tissue section or cell (blood) smear by various antibodies and/or ligands labeled for example with fluorophores including soft bleaching steps in between. Fluorescence signals are recorded by an automated microscope and camera in a pixel-matrix. Besides the fluorophores also other labelings which allow for a precise detection of the epitopes labeled by the ligands or antibodies bond can be employed. Subsequent binarization and computerized analysis of electronically filed image data allow to detect positive and negative coexpressions of multiple epitopes in a pixel-based manner as so-called CMP's.

Some CMP's (or slight variants of them) are extremely common, and appear to define functional regions of the cell or tissue in question. These features can be formally defmed as CMP motifs: sets of CMP's in which: (i) one or more proteins are invariably present (the lead protein(s)); (ii) one or more proteins are never present (absent proteins); and (iii) additional proteins are present in some but not all members of the set (wild-card proteins). Each motif can thus be denoted by a sequence of 1s, 0s, and *s, indicating the lead, absent, and wild card proteins, respectively. By assigning one colour to each (relevant) pixel- or whole cell-CMP motif, a map of the motifs within any given cell or tissue under investigation can be produced, summarizing the protein organization throughout the region termed a toponome map. As protein compartmentalization in cells and tissues determines structure and function, toponome maps make it possible to identify spatially confined functional entities for the first time.

By capturing the spatial arrangement of specific epitopes in a sample using the MELC technology therefore the phenotype specific to the somatic stem cells can be reliably identified. Therefore according to the invention a corresponding method is provided. The in-situ identification of human somatic stem cells realized by means of the invention facilitates their further biochemical and molecular characterization. Moreover, the application of the MELC technology opens a plurality of new approaches in the identification and characterization of somatic stem cells in the following research and application fields: wound healing, tissue replacement, tissue regeneration, bums medicine, transplantation and grafting medicine, genodermatoses, genetically determined diseases, somatic gene therapy, aging processes, anti-aging, tumor stem cells, molecular medicine of the inflammation, cell cycle and apoptosis as well as cell immortalization.

The phenotype "Ki67 *off*/ CD71 *off*/ pan cytokeratin *on*/ CD29 *on*/ CD49d *on",* being specific to keratinocyte stem cells can be comfortably detected using the MELC technology in particular the MELC robot technology. According to the invention therefore a method for identifying keratinocyte stem cells in a tissue sample, in particular in a tissue sample of a patient, is provided, with the spatial arrangement of the epitopes Ki67, CD71, pan cytokeratin, CD29, CD49d captured for identifying phenotype "Ki67 *offl* CD71 *offl* pan cytokeratin *on*/CD29 *on*/CD49d *on*". The identification of this phenotype, CMP or CMP-motif allows for a highly reliably identification of these stem cells and the performance of this method requires a very short period of time, so that the result is immediately available. Advantageously the used tissue sample is a sample of the skin, in particular a sample of the epidermis, and further advantageously it is a section of the skin tissue.

In a further variation of the invention it is provided to capture the spatial arrangement of the epitopes by means of employing in particular labeled antibodies and/or biologics and/or parts of biologic and/or ligands binding specifically to the epitopes. Above all monoclonal antibodies have a very high specificity due to the employment of preparation techniques known to the expert in the field.

A fluorescent dye particularly easy to detect is suitable for labeling. Examples herefor are phycoerythrin or fluorescein. Moreover labeling with quantum dot is possible.

The spatial arrangement of the epitopes may be captured in an advantageous variation of the invention via the sequence of the following method steps: (a) applying a solution to the tissue sample; (b) allowing the solution to take effect and removing the solution; (c) recording an image prior to or after removing the solution; with the solution containing at least one labeled antibody and/or ligand specifically binding to one of the epitopes. This sequence is the core of the already mentioned MELC method which is described in detail in the printed patent specifications US 6,150,173, US 6,924,115, US 6,974,675, DE 197 09 348, EP 0 810 428, EP 1 136 822, and EP 1 136 823 and to which reference is made herewith.

Steps (a) to (c) may also be repeated with at least one further solution which equally contains at least one labeled antibody and/or ligand binding specifically to one of the further epitopes. Moreover possibly subsequent to step (b) and/or step (c) a washing step and/or subsequent to step (c) a bleaching step may be performed. The bleaching step may prove necessary or at least advantageous when employing antibodies and/or ligands coupled to the fluorescent dye.

Particularly preferred for the identification of the phenotype specific to the keratinocyte stem cells is the employment of at least one antibody which is member of the following group:
- 7B11, an in particular fluorescein-labeled antibody which is directed against Ki67,
- YDJ.1.2.2, an in particular fluorescein-labeled antibody which is directed against CD71,
- MNF116, an in particular fluorescein-labeled antibody which is directed against pan cytokeratin,
- 4B7R, an in particular fluorescein-labeled antibody which is directed against CD29,
- 44H6, an in particular fluorescein-labeled antibody which is directed against CD49d.

The combination of letters/numbers mentioned designates the clone from which the respective antibody is obtained. Besides the fluorescein labelings of course other labelings with fluorescent dyes or quantum dot are possible, too. The named antibodies have proven particularly suitable in a number of tests.

In a particularly preferred variation of the invention the MELC robot technology is used for the detection of the specific phenotype.

The above mentioned object is also solved by providing a composition or a kit, which comprise at least one antibody and/or at least one ligand, wherein the antibodies and/or ligands bind the epitopes Ki67, CD71, pan cytokeratin, CD29 and CD49d specifically and are coupled with particularly different labelings each. If different labelings are used, the individual antibodies and/or ligands and thus also the epitopes bound through them can be differentiated in the case of a simultaneously performed labeling. By employing the composition according to this variation of the invention the time required for the identification of the phenotype is considerably shortened and the performance of the above method facilitated.

In a kit the individual antibodies and/or ligands are contained in different receptacles, but are still made available to the user simultaneously. When using such a kit it is not necessary that the labelings of the epitopes are performed simultaneously, so that the antibodies and/or ligands and/or biologics and/or parts of biologics may also be coupled with identical labelings.

In a preferred variation of the invention at least one of the labelings is a fluorescent dye, in particular phycoerythrin or fluorescein, or a quantum dot.

One variation of the invention exhibits a composition or kit, wherein at least one antibody is a member of the following group:
- 7B11, an in particular fluorescein-labeled antibody which is directed against Ki67,
- YDJ.1.2.2, an in particular fluorescein-labeled antibody which is directed against CD71,
- MNF116, an in particular fluorescein-labeled antibody which is directed against pan cytokeratin,
- 4B7R, an in particular fluorescein-labeled antibody which is directed against CD29,
- 44H6, an in particular fluorescein-labeled antibody which is directed against CD49d.

According to the invention also a biochip for the detection of keratinocyte stem cells is provided, wherein on one surface of the chip ligands and/or antibodies binding specifically to the specific phenotype "Ki67 *off*/ CD71 *off*/ pan cytokeratin *on*/ CD29 *on*/ CD49d *on*" are coupled in such a way that their bondability to the motif is sustained.

Additional features and advantages of the invention derive from the following description of several embodiments of the invention.

In a first embodiment skin biopsies from patients suffering from psoriasis vulgaris are analysed and compared to skin biopsies from patients suffering from atopic dermatitis and to skin biopsies from healthy people. Biopsies of representative skin lesions are taken at the time of inpatient admission because of severe disease deterioration after a wash-out period of 4 or 2 weeks for any specific systemic-/UV- or topical treatment. Normal skin donors are not affected by any inflammatory skin or system disease, nor do they use any immunosuppressive systemic medication.

Punch biopsies of 6 mm diameter are taken from affected and normal skin of patients and donors in local anesthesia using a 1% prilocaine hydrochloride solution.

Since the method according to the invention are preferably only performed in the laboratory, the sample-taking preferably is not part of the method according to the invention.

Freshly taken skin biopsies are snap frozen in liquid nitrogen. In brief, the tissue is placed in frozen specimen-embedding medium at room temperature. The specimen is transferred into liquid isopentane pre-cooled with liquid nitrogen and frozen for 60 seconds. Storage is performed at -80 °C.

Tissue sections of 5 µm thickness are prepared at -25°C using a cryotome (Shandon), fixed in acetone at -20°C for 10 min and stored at -20°C for several days or -80°C for longer time intervals until use. Before use, all samples are rehydrated in PBS at room temperature, incubated with normal goat serum for 30 min, and washed again in PBS.

Aspects of MELC technology have been described above. In brief, a slide with a given specimen is placed on the stage of an inverted wide-field fluorescence microscope (Leica or Zeiss) equipped with fluorescence filters for FITC and PE. Fluorochrome-labelled antibodies and wash solutions are added and removed robotically under temperature control, avoiding any displacement of the sample and objective. In each cycle, a pair of antibodies is added; phase contrast and fluorescence images are acquired by a high-sensitivity cooled CCD camera; the sample is washed ten times with PBS and bleached at the excitation wavelengths for 10 min; and post-bleaching phase contrast and fluorescence images are acquired. The cycle is then repeated with the next set of antibodies. Different developmental stages of the MELC system are progressively automated by using proprietary software. The motor-controlled stage of the microscope is used to record several visual fields in each cycle, which are fused to generate panoramic images.

Fluorescence images produced by each antibody are aligned cell-wise using the phase contrast images. Background and illumination faults are then removed by flat-field correction before excluding artefacts by a mask-setting process. Fluorescence signals are set to 0 (= below threshold) or 1 (= above threshold), using an automated threshold setting method validated by human experts. The binarized images are then combined to form a list representing the proteins expressed in each cell.

In this first embodiment of the invention the location of 48 molecules shown in the Table are analysed chosen to detect a) cell types (keratinocytes, dendritic cells, macrophages, T lymphocytes, NK cells, neutrophil and eosinophil granulocytes, endothelial cells); b) extracellular matrix components (collagen type IV, laminin); and c) inflammatory cell functions (cell activation, proliferation, adhesion). In the Table the library of fluorophore-labeled antibodies as established for the MELC analysis of skin tissue is depicted, wherein the antibodies for CD1a and TIA-1 are marked by phycoerythrin and all remaining are marked by fluorescein. The sequel order of the antibodies within the MELC run is designated by their position in the Table. The sources are the following: a - Progen, b-DAKO, c - Immunotech, d - Ancell, e - Beckman Coulter, f - Sigma, g - Serotec, h - Biozol, j - Zymed, k - Euroclone, m - An der Grub, n - Hölzel, p - Becton Dickinson. The antibodies are applied in the dilution documented in the Table. The frequency of pixels positive for each molecule in each tissue compartment (such as epidermis or dermis) is normalized to a horizontal width of 100 µm so as to avoid being influenced by the vertical stratification of epithelial tissue, and tissue compartments are distinguished by positive and negative masks defined largely by means of a pan-cytokeratin antibody (results not shown).

Three-dimensional imaging of MELC runs is performed by generating and visualizing z-stack raw images for each antibody signal from top to bottom of a sample, followed by the steps of: deconvoluting these images using a standard algorithm working with a specific point spread function; setting thresholds for each antibody signal from each optical plane as before; overlaying all binarized images to construct large scale protein colocalization maps using a

**Table:**

| **Nr./No.** | **Epitop/Epitope** | **Klon/Clone** | **Verdünnung/Dilution** | **Nr./No.** | **Epitop/Epitope** | **Klon/Clone** | **Verdünnung/Dilution** |
|---|---|---|---|---|---|---|---|
| 1 | CK17^{a} | Ks17.E3 | 1:10 | 25 | CD29^{h} | 4B7R | 1:10 |
| 2 | CD1a^{b} | NA1/34 | 1:100 | 26 | CD18^{h} | YFC118.3 | 1:10 |
| 3 | CD2^{c} | 39C1.5 | 1:10 | 27 | CD49d^{h} | 44H6 | 1:50 |
| 4 | CD38^{c} | T16 | 1:10 | 28 | CD44^{c} | J-173 | 1:200 |
| 5 | CD16^{c} | 3G8 | 1:10 | 29 | CD49f^{h} | 4F10 | 1:10 |
| 6 | CD62L^{c} | SK11 | 1:10 | 30 | CD10^{c} | ALB2 | 1:10 |
| 7 | CD25^{d} | 7G7B6 | 1:100 | 31 | Ki67^{j} | 7B11 | 1:80 |
| 8 | CD62E^{d} | HAE-1f | 1:100 | 32 | CD45R0^{k} | UCHL1 | 1:50 |
| 9 | CD4^{e} | Coulter T4 | 1:10 | 33 | CD34^{b} | QBEnd10 | 1:100 |
| 10 | CD8^{c} | B9.11 | 1:10 | 34 | MPO^{m} | H-43-5 | 1:50 |
| 11 | HLA-DR^{c} | Immu-357 | 1:50 | 35 | TIA-1^{c} | 2G9 | 1:20 |
| 12 | HLA-DQ^{j} | HK19 | 1:10 | 36 | lamininⁿ | - | 1:50 |
| 13 | CD26^{c} | L272 | 1:10 | 37 | CD30^{b} | Ber-H2 | 1:10 |
| 14 | CD45RA^{c} | ALB11 | 1:10 | 38 | CD68^{b} | KP1 | 1:100 |
| 15 | CD57^{c} | NCl | 1:10 | 39 | CD31^{d} | 158-2B3 | 1:600 |
| 16 | CD54^{c} | 84H10 | 1:10 | 40 | CD94^{d} | HP-3D9 | 1:60 |
| 17 | CD56^{c} | NCAM16.2 | 1:10 | 41 | MBPⁿ | - | 1:50 |
| 18 | CD7^{c} | 8H8.1 | 1:10 | 42 | CD11a^{b} | MHM24 | 1:50 |
| 19 | CD58^{c} | AICD58 | 1:10 | 43 | CLA^{p} | HECA-452 | 1:10 |
| 20 | CD138^{g} | B-B4 | 1:200 | 44 | SMA' | 1A4 | 1:100 |
| 21 | CD13^{c} | SJ1D1 | 1:10 | 45 | pan-CK^{b} | MNF116 | 1:10 |
| 22 | CD71^{c} | YDJ.1.2.2 | 1:50 | 46 | CD3^{c} | UCT1 | 1:10 |
| 23 | CD11b^{c} | 44 | 1:10 | 47 | col IVⁿ | - | 1:1600 |
| 24 | CD36^{c} | FA6-152 | 1:10 | 48 | CD15^{d} | AHN1.1 | 1:40 |

The sequel order of the antibodies within the MELK run is designated by their position in the table.
Sources: a - Progen, b - DAKO, c - Immunotech, d - Ancell, e - Beckman Coulter, f - Sigma, g - Serotec, h - Biozol, j - Zymed, k - Euroclone, m - An der Grub, n - Hölzel, p - Becton Dickinson.
CK = Cytokeratin, MPO = Myeloperoxidase, MBP = Major Basic Protein, SMA = Smooth Muscle Actin, col = Collagen

MATLAB algorithm; and constructing three-dimensional toponome maps in the same way as is done for two dimensions. The latter two visualization steps are performed by using algorithms provided by IMARIS software packages.

Cell-based detection features of MELC robot technology allow to identify stem cells of keratinocytes in situ with the phenotype Ki67⁻/CD71⁻/pan-CK⁺/CD29⁺/CD49d⁺. The number of such cells is somewhat reduced in atopic dermatitis (24±12% of basal epidermal cells) and significantly reduced in psoriasis (11±7%) compared to healthy controls (34±7%), possibly reflecting a disease-induced increase in the proliferative cell fraction.

In a further embodiment of the invention only one tissue sample of a person is examined. Additionally only these antibodies of the Table are employed, which bind specifically to the epitopes Ki67, CD71, pan-CK, CD29 and CD49d. The thereby rendered simplification of the method performed in using all the antibodies listed in the Table results in an enormous time gain. The positive as well as negative identification of the relevant epitopes results in the identification of keratinocyte stem cells in the examined tissue sample. In this embodiment the antibodies are applied also in the dilutions documented in the Table.

The antibodies serving for the identification of the epitopes specific to somatic stem cells may also be combined in a corresponding antibody composition or be comprised as kit.

## Claims

1. Method for identification of somatic stem cells, wherein the spatial arrangement of epitopes in a sample is captured by MELC technology for identifying the phenotype specific to the somatic stem cells.

2. Method according to claim 1, wherein keratinocyte stem cells are identified by capturing the spatial arrangement of the epitopes Ki67, CD71, pan cytokeratin, CD29, CD49d in a tissue sample for identifying the phenotype "Ki67 *off*/ CD71 *off*/ pan cytokeratin *on*/ CD29 *on*/ CD49d *on*", being specific to keratinocyte stem cells.

3. Method according to claim 2, **characterized in that** the tissue sample is a sample of the skin, in particular a section of skin tissue.

4. Method according to claim 2 or 3, **characterized in that** the tissue sample is a sample of the epidermis.

5. Method according to one of the previous claims, **characterized in that** the spatial arrangement of the epitopes is captured by means of employing at least one particularly labeled antibody and/or ligand and/or biologics and/or a part of a biologics binding specifically to the epitopes.

6. Method according to claim 5, **characterized in that** at least one of the labelings is a fluorescent dye, in particular phycoerythrin or fluorescein, or quantum dot.

7. Method according to one of the previous claims, **characterized in that** the spatial arrangement of the epitopes is captured by the sequence of the following method steps:
(a) applying a solution to the tissue sample;
(b) allowing the solution to take effect and removing the solution;
(c) recording an image prior to or after removing the solution;
wherein the solution contains at least one labeled antibody and/or ligand binding specifically to at least one of the epitopes.

8. Method according to claim 7, **characterized in that** steps (a) to (c) are repeated with at least one further solution which also contains at least one labeled antibody and/or ligand and/or at least one biologics and/or at least one part of a biologics specifically binding to at least one of the further epitopes, and
**in that** possibly subsequent to step (b) and/or (c) a washing step and/or subsequent to step (c) a bleaching step is performed.

9. Method according to claims 2 and 5, **characterized in that** at least one antibody is employed which is member of the following group:
- 7B11, an in particular fluorescein-labeled antibody which is directed against Ki67,
- YDJ.1.2.2, an in particular fluorescein-labeled antibody which is directed against CD71,
- MNF116, an in particular fluorescein-labeled antibody which is directed against pan cytokeratin,
- 4B7R, an in particular fluorescein-labeled antibody which is directed against CD29,
- 44H6, an in particular fluorescein-labeled antibody which is directed against CD49d.

10. Method according to one of the previous claims, **characterized in that** the MELC robot technology is used for the detection of the specific phenotype.

11. Composition or kit comprising at least one antibody and/or at least one ligand binding the epitopes Ki67, CD71, pan cytokeratin, CD29, CD49d specifically and being coupled each with in particular different labelings.

12. Composition or kit according to claim 11, **characterized in that** at least one of the labelings is a fluorescent dye, in particular phycoerythrin or fluorescein, or a quantum dot.

13. Composition or kit according to claim 11 or 12, **characterized in that** at least one antibody is a member of the following group:
- 7B11, an in particular fluorescein-labeled antibody which is directed against Ki67,
- YDJ.1.2.2, an in particular fluorescein-labeled antibody which is directed against CD71,
- MNF116, an in particular fluorescein-labeled antibody which is directed against pan cytokeratin,
- 4B7R, an in particular fluorescein-labeled antibody which is directed against CD29,
- 44H6, an in particular fluorescein-labeled antibody which is directed against CD49d.

14. Biochip for the detection of keratinocyte stem cells, wherein on one surface of the chip at least one ligand and/or at least one antibody binding specifically to the specific phenotype "Ki67 *offl* CD71 *offl* pan cytokeratin *on*/ CD29 *on*/ CD49d *on*" are coupled in such a way that their bondability to the phenotype is sustained.

15. Use of the MELC robot technology for in-situ identification of somatic stem cells.
